# EUROPEAN PATENT APPLICATION

(11) **EP 2 645 282 A2**
(43) Date of publication of application: **02.10.2013**
(21) Application number: 13161491.9
(22) Date of filing: 27.03.2013
(51) Int. Cl.: G06F 19/00

(54) **System for use in a network and for storing data imported from one or more distinct devices, and associated method**

(30) Priority: 29.03.2012 GB 201205600
(71) Applicant: Research in Real Life Limited, Norwich Norfolk NR1 1HP (GB)
(72) Inventor: Price, David, Norwich, Norfolk NR1 1HP (GB)
(74) Representative: Vienne, Aymeric Charles Emile

(57) **Abstract**

In one aspect, the invention provides a system (4) for use in a network and for storing data imported from one or more distinct devices (1, 2, 3) of the network in a database (40), comprising a first module (41) running on a first storage device (1) and a second module (42) to import anonymous data from the first storage device (1). In another aspect, the invention provides a method performed on the system. In some aspects, the invention has particular, although not exclusive, relevance to the use of anonymized patient data for health research and patient care improvement.

## Description

### FIELD OF THE INVENTION

The invention relates to a system for use in a network and for storing data imported from one or more distinct devices of the network in a database. The invention has particular, although not exclusive, relevance to the use of anonymized patient data for health research and patient care improvement.

### BACKGROUND OF THE INVENTION

Major developments in Information Technology have resulted in significant improvements in the way patient data is recorded in computer systems by General Practitioners (GP) or nurses on Primary Care premises (i.e. the principal points of consultation for patients). This has enabled routine patient data to be used for a variety of population-based research, ranging from epidemiological to drug effectiveness studies.

Primary Care Informatics involves the study of patient data, including medical history and consultations to target key clinical areas and improve health care. The importance of Primary Care Informatics has increased in parallel with the above mentioned improved computerisation of Primary Care and the consequent availability of large volumes of routinely collected longitudinal data.

The advent of standardised data extraction tools such as Morbidity Information Query and Export Syntax (MIQUEST) has enabled these data to be aggregated into large databases with considerable research potential. For example, the General Practice Research Database (GPRD) emphasises the amount of the patient data held in their warehouse and the systematic way it is collected. Thus, the GPRD is often referred to as a standard for research databases.

However, the use of routine patient databases for conducting research and providing improved patient care is still an emerging discipline, and main issues arise at least regarding both the relevance of the data in the databases and the importation of confidential patient data in the databases.

### SUMMARY OF THE INVENTION

Aspects of the present invention address or at least ameliorate at least one of the above issues.

According to one aspect, the invention provides a system for use in a network and for storing data imported from one or more distinct storage devices of the network in a database, comprising:
a first module configured to
   search data stored in a first storage device comprising a memory storing a data bank comprising sets of data associated with users, the search being performed as a function of criteria to identify at least a user and an associated set of data, both corresponding to the search criteria,
   remove personal information about the identified user from the set of data to generate an anonymous set of data,
   allocate a first identifier to the user, and link the anonymous set of data to the first identifier;
a second module configured to
   import the anonymous set of data and the first identifier from the first storage device, generate an anonymous set of requests for information to be sent to the identified user, as a function of the anonymous set of data, and link the anonymous set of requests for information to the first identifier,
   provide the anonymous set of requests for information to an output device,
   import an anonymous set of responses from responses to the anonymous set of requests sent back by the user through a second storage device comprising a memory, and link the anonymous set of responses to the first identifier,
the system further comprising a database, and
wherein the second module is further configured to provide:
   the anonymous set of data and the first identifier from the first storage device and
   the anonymous set of responses from the second storage device
to the database, and
the database is further configured to store the anonymous set of data and the anonymous set of responses, and link them to the first identifier.

The second module may further be configured to generate an anonymous status as a function of at least the anonymous set of data and the anonymous set of responses from the database, and link the anonymous status to the first identifier, and generate a summary as a function of a plurality of anonymous statuses from the database. The database may be configured to store the anonymous status and the summary. The second module may further be configured to provide the anonymous status and the summary to the first storage device, and the first module is configured to link back the anonymous status to the identified user thanks to the first identifier, to generate a personalized status. The database may further be configured to allocate a second identifier to the first identifier in a first database, and store the second identifier, the anonymous set of data and the anonymous set of responses in a second database, the database linking the anonymous set of data and the anonymous set of responses to the first identifier via the second identifier.

The first module may further be configured to provide personalized information about the identified user to the output device.

The output device may comprise at least one of a printer and a server. The output device may comprise a printer configured to print the anonymous set of requests for information to be sent to the identified user as a questionnaire comprising the first identifier, and the personalized information as a cover letter comprising pieces of personal information about the identified user.

The second storage device may comprise at least one of a scanner and a server. The second storage device may comprise a scanner configured to scan the anonymous set of responses, from the identified user and comprising the first identifier, as a questionnaire filled in by the identified user, using an optical character recognition software.

The output device may further comprise the second storage device and comprise a server which is configured to be accessed by a graphical user interface to display the anonymous set of requests to the identified user and to enable the identified user to enter the set of responses to the anonymous set of requests.

The second module may further be configured to import a set of result data regarding measures concerning the identified user from a third storage device, and link the set of result data to the first identifier, and provide the set of result data from the third storage device to the database. The database may further be configured to store the set of result data, and link them to the first identifier. The second module may be configured to perform a verification of the imported data from the first device, or to perform a case-control matching on the data stored on the database.

The third storage device may comprise an adapter to an inhaler belonging to the identified user and/or an acceleration tester.

According to another aspect, the invention provides a method performed by a system in a network, for importing data from one or more distinct storage devices of the network, and for storing the imported data in a database, the method comprising the steps according to which: a first module
searches data stored in a first storage device comprising a memory storing a data bank comprising sets of data associated with users, the search being performed as a function of criteria to identify at least a user and an associated set of data, both corresponding to the search criteria,
removes personal information about the identified user from the set of data to generate an anonymous set of data,
allocates a first identifier to the user, and links the anonymous set of data to the first identifier;
a second module
imports the anonymous set of data and the first identifier from the first storage device,
generates an anonymous set of requests for information to be sent to the identified user, as a function of the anonymous set of data, and links the anonymous set of requests for information to the first identifier,
provides the anonymous set of requests for information to an output device,
imports an anonymous set of responses from responses to the anonymous set of requests sent back by the user through a second storage device comprising a memory, and links the anonymous set of responses to the first identifier,
the method further comprising the step wherein
the second module further provides:
the anonymous set of data and the first identifier from the first storage device, and
the anonymous set of responses from the second storage device
to a database, and the database stores the anonymous set of data and the anonymous set of responses, and links them to the first identifier.

The second module may perform a case-control matching on the data stored on the database.

The system may identify matching criteria; create a combined database file containing records for patient cases, corresponding control patients and identified matching criteria variables; randomly select from the combined database file a case-control pair, where a case matching criteria variables match a control matching criteria variables, and write the case-control pair record in a matching table file, select a case-control matching ratio, and randomly select a case identifier and randomly select at least a control identifier matched to the case identifier from the matching table file; and write the matched case and control in a holding data table file.

The search criteria to identify at least a user and an associated set of data may be the matching with a particular disease group comprising at least one of chronic obstructive pulmonary disease, COPD, and asthma.

The user may be a patient registered with a general practitioner, and the sets of data may be clinical data records.

According to one or several other aspects, the invention provides a computer program, a signal, a computer program product or a computer readable medium comprising instructions for carrying out a method according to aspects of the invention.

### BRIEF DESCRIPTION OF THE DRAWINGS

Embodiments of the invention will now be described, by way of example, with reference to the accompanying drawings, in which:
- Fig. 1 is a schematic diagram of a system according to aspects of the invention;
- Fig. 2A and 2B are schematic diagrams of an example of a method according to aspects of the invention;
- Fig. 3 is a schematic diagram of records and identified records stored in a memory of a first device of Fig. 1,
- Fig 4 is a schematic diagram of an anonymous identified record and anonymous self-completed questionnaire stored in a memory of the system of Fig. 1,
- Figs. 5 is a schematic diagram of an example of a case-control matching performed by the system of Fig. 1, and
- Fig. 6 is a schematic diagram of an example of tables created during a case-control matching of Fig. 5.

In all the figures, similar components bear identical numerical references.

### DETAILED DESCRIPTION OF THE INVENTION

### Overview

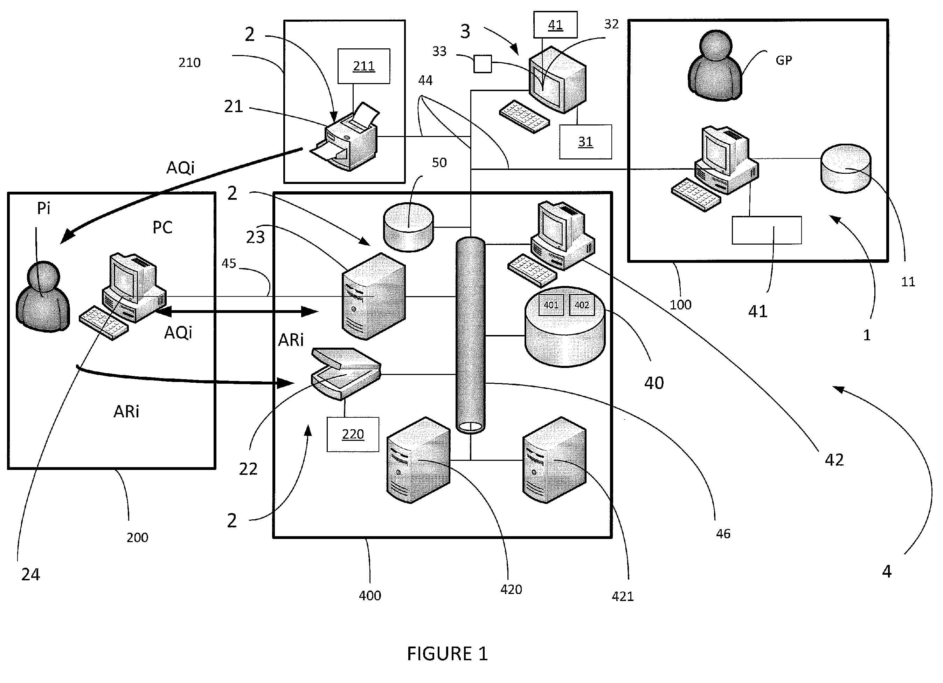

Referring now to the drawings, Fig. 1 shows a system 4 for use in a network comprising one or more distinct devices referred to as 1, 2 and 3.

The first device 1 comprises a computing and management system of a General Practitioner (GP), located on the premises 100 of the GP's practice, commonly referred to as a NHS Primary Care Practice. The system may be a personal computer 1, and comprises a memory 11 storing GP's patient clinical data forming a data bank. The data bank comprises records of clinical data (such as morbidity and prescription data) associated with the patients of the GP.

Although not represented in Fig. 1, a plurality of GPs are linked between each others by a national broadband network for the English National Health Service (NHS), also referred to as N3. N3 is a secure broadband network enabling the provision of several services, such as the NHS Care Records Service.

The system 4 comprises a first module 41, running on the computer 1. The first module 41 is configured to be able to perform searches in the data bank stored in the memory 11 of the computer 1. The search can be performed as a function of several criteria, to identify one or more patients Pi (with i=1...n, n being any positive natural integer) corresponding to the search criteria. In this example, the identification of the patients is performed based on particular disease-specific read codes (i.e. the standard clinical terminology system used in General Practice in the United Kingdom) used by the NHS and/or prescription information in the records. The first module 41 is configured to perform Morbidity Information Query and Export Syntax (MIQUEST) queries in the data bank of the memory 11, to identify and extract patient's relevant morbidity and prescription data.

The identification of the patients may be performed periodically, at regular intervals of time.

The system 4 also comprises a second module 42, located at a location 400 remote from the premises 100 of the GP's practice. The second module 42 is configured to perform a File Transfer Protocol (FTP) through a communications network 44 linking the system 4 and the computer 1. As described below in further details, the module 42 is configured to import data from the personal computer 1, process the imported data and provide the processed data to a database 40 of the system 4, via a secure network 46 on the premises 400.

The database 40 is configured to store the processed data, so that the data is available for further processing, such as patient care improvement and health research. The system 4 may also comprise a database 50 dedicated to studies, as well as the main database 40. The data stored on the database 50 may be coded for individual research studies, to ensure that coding terms and information data are captured in an effective and consistent manner to facilitate meaningful interpretation and analysis.

Although not represented in Fig. 1, the system 4 may be connected to a plurality of GP's computers 1 through the communications network 44. In an example, the communications network 44 is at least partially comprised in the national broadband network N3, or the system 4 can access the N3 via access points. Several hundreds of practices may thus be linked to the system 4, and contribute to a growing research database with patient information as explained further below.

The network further comprises one or more second storage devices 2, distinct from the computer 1. Each of the second devices 2 may be a data output device and/or a data storage device.

In an example, an output device 2 may comprise a multifunction peripheral 21 comprising a memory 211. The peripheral 21 is located in a secure printing facility 210, remote from the GP's practice 100 and also from the premises 400. The peripheral 21 is however connected to the first module 41 through the communications network 44, and the memory 211 is configured to store data (such as anonymous questionnaires AQi) received from the module 41 in an electronic format as explained below. The peripheral 21 is preferably an industrial printer for printing paper data from the electronic data received from the first module 41. In an example, paper questionnaires AQi are to be sent to the patients Pi identified by the first module 41. To that effect, the peripheral 21 is configured to automatically place the printed paper questionnaires AQi in envelopes (a personalized cover letter and a freepost envelope addressed to the premises 400 may also be placed in each envelope), seal the envelopes and post the sealed envelopes to the identified patients Pi. Therefore the GPs do not need to send any questionnaire themselves to the patients Pi, and the required amount of work from the GP is therefore minimised. In an example, the peripheral 21 is an NHS approved facility, and in order to enable the provision of the questionnaires AQi to the peripheral 21 via the network 44, access to the necessary NHS national gateway firewall ports is provided to the system 4.

In another example, a storage device 2 may comprise a scanner 22 configured to scan the self-completed paper questionnaires returned by the patients Pi, extract responses ARi using an Optical Character Recognition (OCR) software, and store the responses ARi in a memory 220 of the scanner 22. In an example, the scanner 22 is located in the premises 400.

In another example, an output device 2 may comprise a server 23 located in the premises 400 and storing the questionnaires AQi in an electronic format. The server 23 is linked to computers 24 of the patients Pi, located in the premises 200 of the patients Pi (private house) or in the premises 200 of a dedicated place (such as a public health centre), via a communications network 45. Although not represented in Fig. 1, the server 23 may be accessed online by a plurality of computers 24 through the communications network 45, such as the Internet. Each computer 24 comprises a user interface, and in this example, the user interface comprises a graphical user interface to display the questionnaire AQi in an electronic format to the identified user Pi. The graphical interface of the computer 24 allows the identified user Pi to enter responses ARi to the questionnaire AQi in an electronic format. The server 23 is also configured to store the responses ARi.

In the example of figure 1, the network also comprises a third storage device 3. In this example, the device 3 comprises an inhaler and/or acceleration technique assessment device 3, configured to store assessment results from an adapter 33 to an inhaler of a patient or an acceleration tester 33, in a memory 31. The device 3 is linked to the system 4 via the network 44. The first module 41 can also be run on the device 3, at least for anonymization purposes.

The networks 44 and 45 may comprise any type of network connections, for instance ADSL or cable connections or wireless connections.

### First module

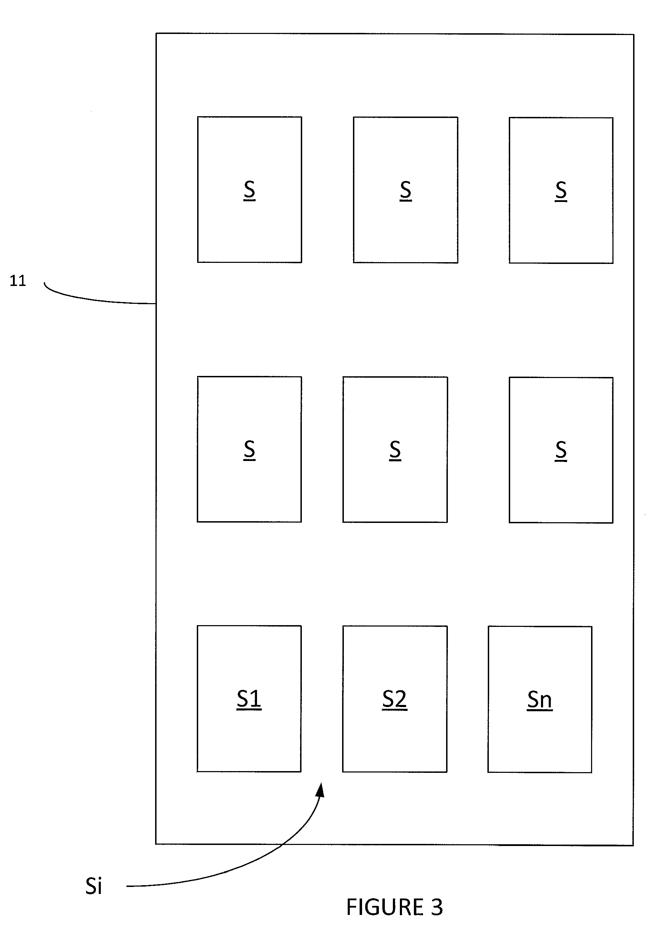

As stated above and as shown in Fig. 3, the first module 41 is configured to perform Morbidity Information Query and Export Syntax (MIQUEST) queries in the data bank of the memory 11 storing the patient records S, to identify and extract patient's relevant morbidity and prescription data. The identified records are referred to as Si (with i=1...n).

The module 41 is also configured to anonymize the identified and extracted data Si, by removing all personal information (such as names and addresses) in the identified and extracted records, to generate anonymous records in an electronic format, referred to as ARi (with i=1...n). The anonymization performed by the first module 41 adopts architectures, technologies, and cryptography systems known to the skilled in the art, and refrains from combining data with the intention of identifying the patients. The module 41 is also configured to allocate a unique identifier αi to each patient Pi, and also to each data extraction.

The module 41 is also configured to generate an anonymous questionnaire AQi to be sent to the identified user Pi, the generation being performed from the anonymous record ASi. The anonymous questionnaire AQi is a request sent via an output device, for a response from the identified patient, via a second data storage device. The response contains the requested data, for completing the data of the database. The system 4 can therefore improve the completeness of the data of the databases, and the relevance of the data in the database for improving the patient care.

### Second module

The second module 42 is configured to import data from each computer 1, for clinical review and research purposes. The second module 42 is configured to upload the extracted data from the computer 1, via a FTP, to a secure off-site file server 420, and to transfer the uploaded data from the server 420 to a secure data server 421 for further processing. The module 42 is also configured to verify the quality of the transferred data, via a verification process, prior to importation of the verified data into the database 40. The module 42 performs the verification process using systematic and detailed check of the context in which the data were recorded. The check enables to conduct robust assessment of the validity of all data and to sort out the data which is sent to the database 40. The verification performed by the second module 42 ensures that the database 40 stores quality data.

In an example, the module 42 is configured to check an extract number, to ensure it is in sequence with the previous extract for said computer 1. This check ensures that there is no gap in the data, and therefore that no data has been lost or corrupted. In another example, the module 42 is configured to perform a data preparation for the storing in the database 40, such as a comma-delimitation of the data, with all fields enclosed in double-quotes (ASCII code 34) and with all non-printing ASCII characters (codes 0-31, 127-255) moved. As a result all files have a header line where the field names are comma-delimited and enclosed in double quotes. The module 42 may also be configured to check that the prescribed data formats are in accordance with the data type. The module 42 can also check the data which are defined as mandatory for the database's integrity and which must be updated regularly (such data may be at least one of drug product lists, clinical read code lists, prescribing instructions cross reference lookup table), to ensure that there is no null string for these data, which would put the database integrity at risk. The module 42 may also check the length of the anonymous patient identifiers. The module 42 is also configured to checks if criteria regarding the referential integrity and the patient consistency, as listed below, are met:
As regards to referential integrity, the criteria of the check may be at least one of the following:
   Valid practice record;
   Valid registration date of the patient;
   Appropriate terminator code for the clinical read codes;
   Match of the therapy drug codes with a multiplex product lookup table; Match of the prescribing instructions with a cross-reference instruction lookup table;
As regards to the patient consistency, the criterion may be a match of the patient record with an existing record for that practice. The patient matching in the practice is based on the following:
   anonymous patient id,
   gender,
   year of birth, and
   practice registration date.

If a patient is not matched on all criteria, the module 42 creates the patient as a new patient.

For all inconsistencies in the data, the module 42 issues an error message and a report detailing the reasons for the non-importing of the patients details in the database 40. The message and report are sent to the first module 41, so that the first module 41 can send a recommendation and/or a request for update and/or correction of the corresponding data to the computer 1, for displaying to the GP. The system can therefore improve the completeness of the data of the databases. The messages and reports can be stored in the computer 1 and/or the system 4 for audit purposes.

### Database

Data stored in the database 40 is organised according to the following structure.

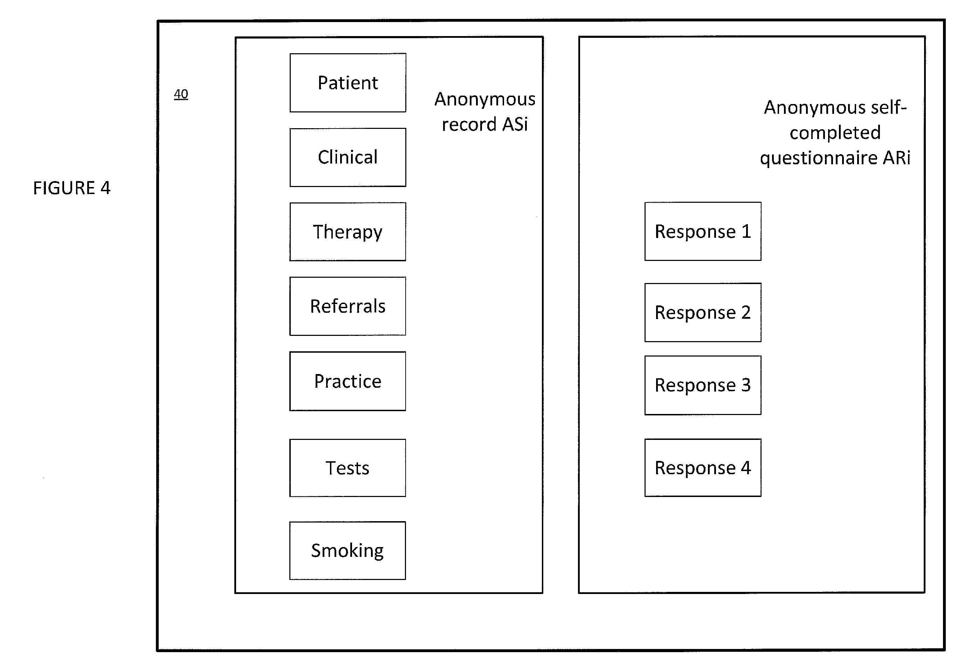

Patient-level information held in the database 40 comprises non-linked, anonymous data. All data held in the database 40 is held in strictest confidence and the need for confidentiality takes precedence over considerations of economy or convenience. For each importation of data, the number of records in the database 40 must match the numbers of records already in the database 40. As shown in Fig. 4, each record stored in the database 40 consists of:
Anonymous, routinely collected data ASi derived from periodic extractions from the computers of the practices and undertaken for clinical review;
Anonymous data from patient self-assessment responses ARi in the questionnaires collected as part of the clinical review.

The system 4 is therefore adapted to manage importation of data from different data sources (i.e. the distinct storage devices).

The anonymous records ASi contains the following sections:
Patient,
Clinical,
Therapy,
Referrals,
Practice,
Tests, and
Smoking.

Each section of the record contains one or more subsections, as follows.

The patient section contains the following demographics subsections:
Date of birth (year only), and
Gender.

The clinical section contains the following subsections:
Medical history data pertaining to a selection of clinical records, defined by a compiled list of read codes.

In an example, the medical history data pertain to two particular disease groups, i.e. chronic obstructive pulmonary disease (COPD) and asthma.

The therapy section contains the following subsections:
Drugs prescriptions data, for all the drugs prescribed by the GP.

Multiple rows of data are allowable per patient. Drug codes are based on Multiplex and British National Formulary (BNF) codes.

The referrals section contains the following subsections:
Data about all referrals for the identified patients, and
Reason for referral.

The referrals are identified by a medical code indicating referral to external care centres (normally Secondary Care locations, such as hospitals for in-patient or out-patient care). Multiple rows of data are allowable per patient.

The practice section contains the following subsections:
NHS Regional Health Authority code.

The tests section contains the following subsections:
Records data of all respiratory system-related tests and investigations for the defined identified patients.

The data is coded using a Read code which will mostly identify the type of test used. Patients may have more than one row of data.

The smoking section contains the following subsections:
Current and historic records data of smoking details for the identified patients. Patients may have more than one row of data.

### Inhaler/Acceleration technique assessment device

The device 3 comprises an adapter 33 to an inhaler belonging to the identified patient Pi and/or an acceleration tester 33. In an example, the adapter and the acceleration tester are combined, to assess the acceleration of the air and the duration of the inhalation when the patient is using his inhaler for treatment. The tester and/or adapter 33 are linked to a computer 32 for processing results Mi of the tests as regards to COPD and/or asthma. The asthma and COPD identified patients are individually assessed using the device 3. Nurse support may be provided to conduct in-depth asthma reviews. The device 3 enables therefore improving asthma control, via a structured inhaler technique assessment, to evaluate handling of the own inhaler of the patient Pi and/or inhalation or acceleration technique, in relation to optimum thresholds and recommendations. This might result in suggesting at least one of altering the patient's therapy or inhaler and improving the patient's technique.

The results of the tests are stored in the memory 31, and are imported from the memory 31 by the second module 42, preferably once an optional first module 41 running on the third device 3 has anonymized the result data Mi regarding the measures. The result data Mi are linked to the identifier in the database 40, preferably in an anonymized fashion.

The device 3 may be located in the premises 100 of the practice, or in another location remote from the premises 400. The system 4 is therefore adapted to manage data importation of data from different data sources.

### Operation

### Clinical review

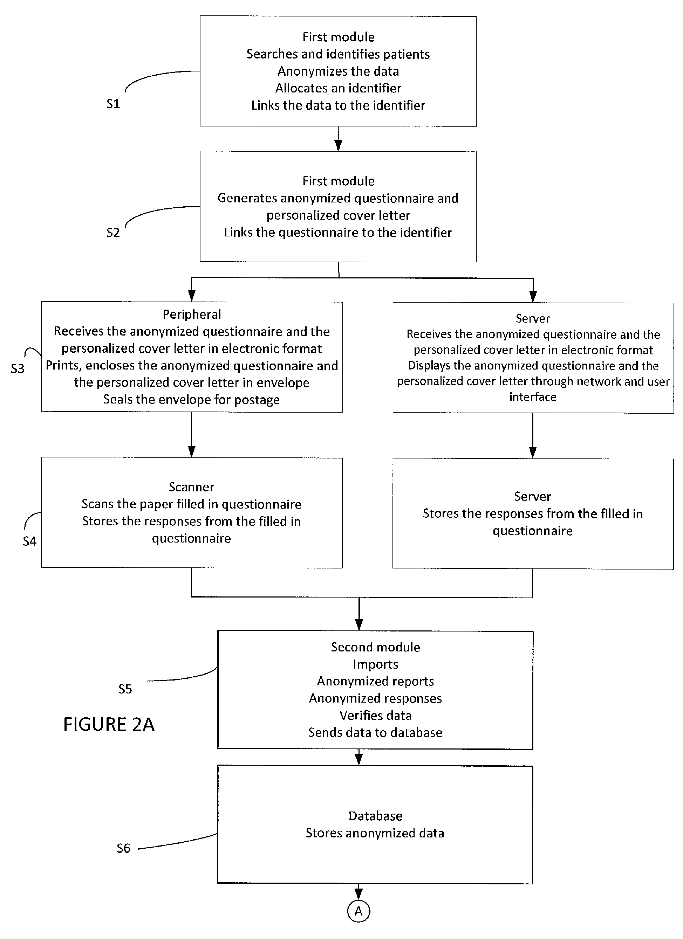

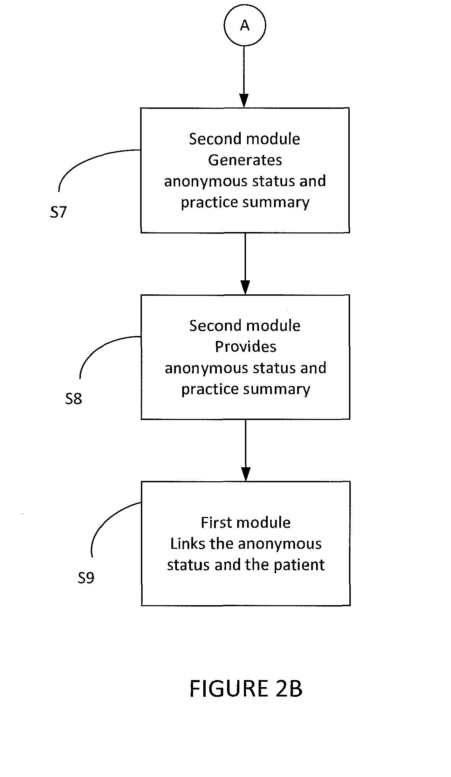

Fig. 2A and 2B show diagrams illustrating an exemplary method performed by the system of Figure 1.

In S1, the first module 41 runs a search in patients records S stored in the memory 11 of the PC 1 of the GP. The search includes MIQUEST queries to identify patients Pi (with i=1, 2... n) and their record Si (with i=1, 2... n), in this example, patients matching asthma and/or COPD criteria.

The module 41 may establish a list of the identified patients Pi which may be reviewed by the GP and/or an appropriate clinician, to ensure that only suitable patients Pi are contacted for the clinical review by questionnaire as explained below.

Once the patients Pi and records Si are identified and preferably reviewed, the module 41 removes all personal information about the identified patients Pi (i.e. name, address, post code) from the record Si, to generate an anonymous record ASi. The record ASi comprises the patient's relevant morbidity and prescription data, in an anonymous format, i.e. once the anonymization is performed, the anonymous record ASi only comprises not patient-identifiable data.

Also, the module 41 allocates a first identifier αi to the patients Pi, and links the anonymous record ASi to the first identifier αi.

In S2, the first module 41 generates an anonymous questionnaire AQi to be sent to the identified user Pi from the anonymous reports ASi. The first module 41 links the anonymous questionnaire AQi to the first identifier αi. The first module 41 also generates the necessary personalized cover letters to the identified patients Pi (one personalized cover letter is associated with an anonymous questionnaire AQi). In S2, the questionnaires AQi and the personalized cover letters are in an electronic format.

In S3, the first module 41 provides the questionnaires AQi and the cover letters to the peripheral 21, via the network 44. The questionnaires AQi and the cover letters may be provided in a secure fashion, using an encryption protocol. The peripheral 21 automatically prints the paper questionnaires AQi and the personalized cover letter, encloses the questionnaires, and preferably a freepost envelope, in an envelope, and seals the envelope. Only sealed envelopes leave the facility 210 for postage, for confidentiality reasons.

The identified patient Pi receives the sealed envelope containing the cover letter, the anonymous questionnaire AQi (and the freepost envelope), fills in the questionnaire with adequate responses ARi, and returns the self-completed anonymized questionnaires ARi by post (preferably using the provided freepost envelope) to the premises 400 containing the scanner 22.

In another example, in S3 the first module 41 provides the questionnaires AQi to the server 23 which can be accessed by the patient's PC 24, via the network 45.

The patient Pi receives the personalized cover letter (for instance in an email) and fills in the questionnaire AQi online.

Preferably, an explicit agreement is sought to the patient Pi, so that the anonymous data stored by the database may also be used for research purposes.

In S4, the scanner 22 scans the completed questionnaires ARi using the OCR software and stores the responses ARi in the memory 220 of the scanner.

In the other example involving the server 23, the server 23 stores the responses ARi provided online.

In S5, the second module 42 imports:
the anonymous reports ASi linked to the first identifier αi from the computer 1, and
the anonymous responses ARi from the memory 220 and/or the server 23.

The system 4 therefore manages importation of data from different types of data sources. Also, thanks to the anonymization of the reports by the first module 41, only not patient-identifiable data leaves the GP's practice premises 100. Preferably, written approval is obtained from the GP prior to any reports being imported from each practice 100. Furthermore, thanks to the anonymization of the questionnaires by the first module 41, only not patient-identifiable data leaves the patient's premises 200.

In S5, the imported data are validated and verified by the second module 42. The second module 42 provides the anonymous set of data ASi and the first identifier αi to the database 40.

In S6, the database 40 stores the anonymous reports ASi and the anonymous responses ARi, both linked to the first identifier αi. As a result, in S6, the database 40 stores cross-sectional information (i.e. information for one or more patients) and longitudinal information (several types of information for each patient) about the identified population of patients, i.e. the population impacted by COPD and/or asthma.

Preferably, in S6, the database 40 randomly allocates a second identifier βi to each first identifier αi whilst storing the provided data in a main database 402 of the database 40, to ensure that each patient record ASi is fully anonymous, and has no means of linkage back to the original provided data file held in an interim database 401 of the database 40. The link between the second identifier βi and the first identifier αi is located in the interim database 401, and the database 40 stores the second identifier βi, the anonymous set of data ASi and the anonymous set of responses ARi in the database 402, the database 40 linking the anonymous set of data ASi and the anonymous set of responses ARi to the first identifier αi via the second identifier βi. Each time the data in the main database 402 needs updating, the entire patient database is deleted and replenished using data stored in the interim database 401 to which validation methodologies have been applied. This update guarantees robustness of data stored in the database 40.

In S7, the second module 42 combines data ASi and ARi to generate an anonymous status ASSi, and a practice summary SM. The database 40 stores the anonymous status ASSi and the summary SM.

The anonymous status ASSi represents a profile of COPD and/or asthma health status and reasons for poor health status for the patient Pi. The status ASSi helps identify priorities for review and specialist referral. The status ASSi also assists in informing about the content of the review, which may include at least one of the following:
overall COPD and/or asthma health status, based on relevant clinical data;
current treatment;
potential reasons for suboptimal disease status, and
recommendations for action with considerations.

The potential reasons for suboptimal disease status may comprise at least one of the following:
continued smoking;
poor adherence to prescribed treatment; and
inadequate treatment.

The recommendations for action with considerations may comprise at least one of the following:
modification of existing treatment in line with current guidelines,
smoking cessation, and
referral to respiratory specialist.

The practice summary SM provides targeted advice to the GP, and recommendations on disease management. The summary SM provides also an overview of the extent of COPD and/or asthma across the practice, and identifies the number of patients recommended for review or referral. It may also include data on response rate, COPD and/or asthma population, and a status together with summary information on at least one of the following:
number of patients with optimal COPD and/or asthma health status,
number of patients recommended for in-house review (on the basis of suboptimal status),
number of patients recommended for consideration for specialist referral because of high risk status.

In S7 data Mi from the third device 3 may also be imported from the memory 31 of the third device 3 and compiled by the second module 42 with the ARi to generate the status ASSi. The importation and the compilation of the data may also be performed before, for instance before the generation of the questionnaires.

In S8, the second module 42 of the system 4 provides one or more statuses ASSi and the summary SM (both patient-anonymous) to the computer 1 in the practice premises 100, over the network 44. As an example, the second module 42 provides the statuses and the summary SM to the GP by email, for example within four weeks of questionnaires AQi being mailed out from the premises 210.

In S9, the first module 41 de-anonymizes the statuses ASSi within the practice 100 and links back the status ASSi with the patient Pi thanks to the first identifier αi, to generate a personalized status PSi. The patient's identity to which the status ASSi relates to can only be established at the practice's premises 100, where the first identifier αi is translated back to the patient's identity by the first module 41.

### Research

The data stored on the database 40 may be accessed for research purposes, and be a resource for the research community. The resource is set up as a supported access resource rather than as an open access resource. This requires researchers to submit a proposal describing their research and study protocols. After successful review, the access to the data is approved and each researcher may sign a declaration regarding data protection. Costs for provision of datasets may be determined on a project-by-project basis.

During the study, the module 42 codes the data of the database 40 using the protocols determined by the researchers, and the relevant coded data for the study is uploaded to the study dedicated database 50. The study may be a case-control study, to identify factors that may contribute to a medical condition, by comparing subjects who have that condition (the 'cases') with patients who do not have the condition but are otherwise similar (the 'controls').

### Case-Control Matching

In real world studies as those performed using the database 40, case-control matching may be necessary to ensure similarity of patients included in an analysis, so as to minimise any possibility of outcome confounding, e.g. confounding by severity differences between treatment arms.

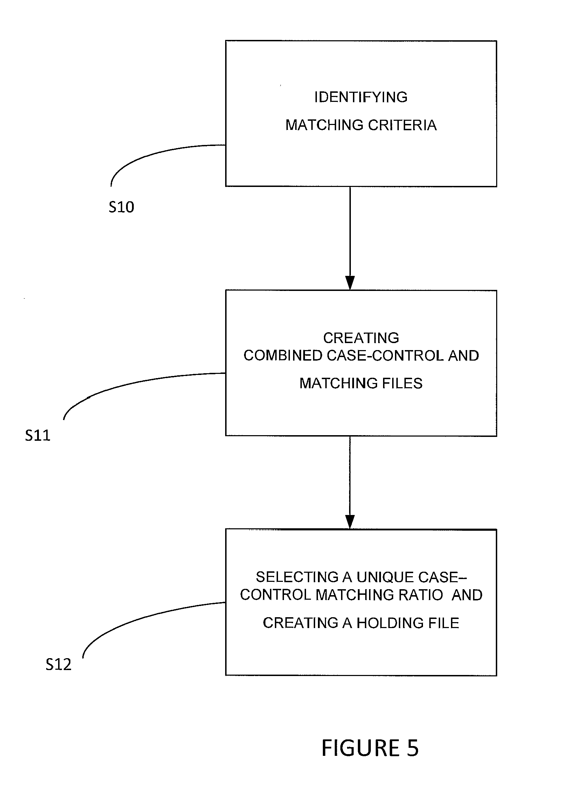

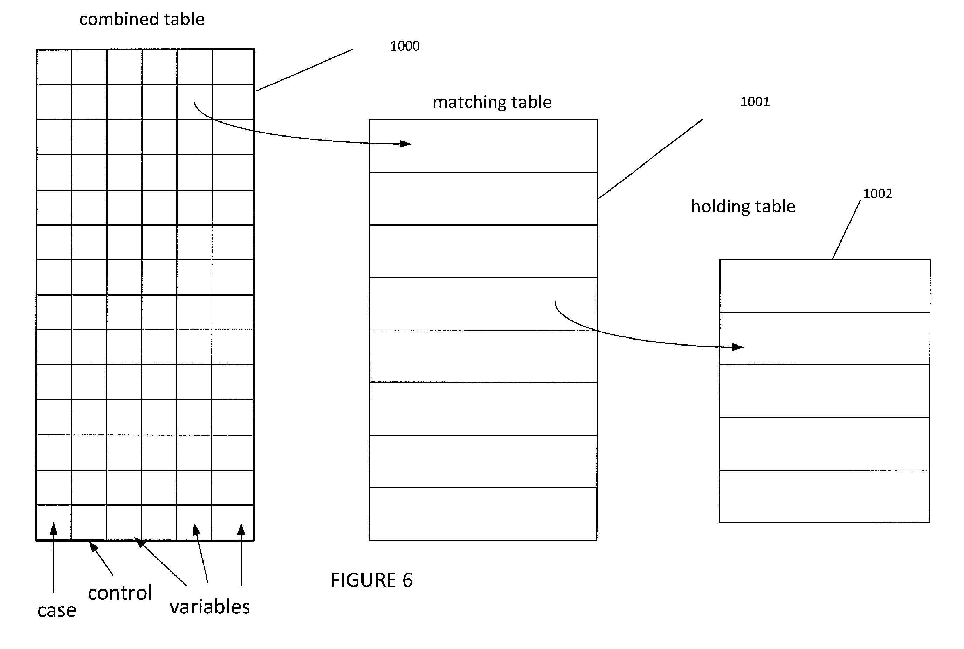

As can be seen in Figs. 5 and 6, the system 4 performs a case-control matching.

In S10 the system 4 identifies the matching criteria, e.g. those where substantial differences are seen between patient groups and those that may result in clinically meaningful differences. Clinical experts may be consulted at this stage to identify any differences that may not appear to be statistically significant but could have important clinical implications. The matching criteria may be a particular criterion, a range, or a relevant category. The matching may be performed:
definitely on a particular criterion (e.g. Male: yes/no), or
within a range (e.g. Age ±x years), or
within relevant (as identified from the baseline population characteristics) categories (e.g. within daily dosing categories for a particular drug or a drug class).

Exact matching enables maximising similarity of patients included in the study analysis, whilst matching within ranges and categories enable improving similarity of patients with retaining greater patient numbers. A quasi-scientific/pragmatic approach should be taken that ensures patient similarity is achieved without losing study power.

In S11, the system 4 creates, for instance in the database 50, a combined database table file 1000 containing a record for each case (i.e. the identified patient having a condition) and joining a control (i.e. a patient not having the condition) to each case. The number of controls will determine the number of rows per case which needs to be created, effectively marrying each case with every control.

Using the control identifier on each record, the system 4 extracts from the controls data file all the previously defined matching criteria variables, and adds these as new columns of data to the combined case/control data file. As an example, if a study has 200 cases and 500 controls, the combined data file contains 100,000 rows of patient identifiers and corresponding control variable information.

The system 4 then links the case identifier from the combined data table file 1000 to the case data record, and begins comparing each corresponding variable between the case variables and the control variables held on the combined file.

As each case could possibly have multiple matched controls, but from which only one unique case-control pairing needs to be randomly selected, once matched, the case-control record is written to a matching table file 1001 to ensure only valid matches are taken forward to the next stage of the matching process.

In S12, the system selects a unique case-control matching ratio.

In an example, the ratio may be a 1:1 ratio. The system 4 loops through the matching table file 1001 and randomly selects a case identifier from all the matched cases limiting the selection to one case at a time, using for instance a well-known SQL function RAND(). The system then loops through the matched control identifiers for that selected case, and randomly selects one control identifier matched to that case.

After a selection has been made, the case and its matching control are written to a holding data table file 1002.

The system 4 then removes every other instance of the case identifier and control identifier from the bulk matching table 1001, which ensures neither the case nor controls could ever be randomly selected again.

When all possible available matches have been made, the final holding data table file 1002 has only those rows where one unique case could be matched to one unique control.

The system 4 assigns a unique sequential identifier to each matched pair and this number is written to the case and controls' actual study data record for further analysis.

As an alternative, the system 4 selects a unique 1:2, 1:3 (or more), case-control matching ratio by randomly selecting a required number of control identifiers available for one case. The final data file has only those rows where one unique case could be matched to the required number (2 or 3 in the example) of unique remaining controls. As the system 4 might not have been able to allocate the specific number of controls every time for a case, a count of the number of controls needs to be made to ensure that for final matching selection, only those cases which have the exact 1:2 or 1:3 ratio are allocated for the study data records.

If insufficient data for the study is stored in the database, the system 4 may send a request for response to the devices 1, 2 or 3. The response may therefore contain the requested data, and therefore complete the data stored in the database for a better relevance of the data in the database for both research and patient care.

### Advantages

The system has numerous advantages. Examples of advantages are listed below.

The system 4 helps Primary Care to produce high quality patient profiles to facilitate efficient clinical review and to optimise treatment to meet the needs of individual patients.

The system 4 also helps GP practices to meet NHS requirements in the audit and care of their patients.

The system 4 also enables research to be conducted on the imported data. Efficient case-control matching or pairing can also be performed.

The extracted patient data are transferred and stored in a confidential fashion.

The system can send requests to devices to complete the data of the databases.

The system manages the importation of data from a diversity of data sources.

### Modifications and Alternatives

Detailed embodiments have been described above. However, the above embodiments are to be understood as illustrative examples of the invention. As those skilled in the art will appreciate, a number of modifications and alternatives can be made to the above embodiments whilst still benefiting from the inventions embodied therein, and further embodiments of the invention are envisaged. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

In the above embodiments, computer systems were described. As those skilled in the art will appreciate, the techniques described in the present application can be employed in other systems, such as, for example, personal digital assistants, mobile phones, laptop computers, web browsers, etc.

In the embodiments described above, the modules may be formed where appropriate, by dedicated hardware, software, firmware or any combination thereof. A software implementation may however be preferred to facilitate the updating of the functionality of the modules.

It should of course be appreciated that, although not explicitly shown in the Figures, the modules 41 and 42, the servers 23, 420, and 421 and the databases 40, 401, 402 and 50 are functional block diagrams and that in practice the individual blocks shown in the Figures may exist as discrete elements or as a single element, or their functionality may be distributed or not individually discernable. As an example, the modules 41 and 42 may or may not be separate software modules, and the database 40 and the memory 11 may or may not be separate storage means.

Where software modules are provided, they may be provided, as appropriate, in compiled or un-compiled form and may be supplied to the computers, as the case may be, as a signal over a computer or telecommunications network, or on a computer storage medium such as for instance a CD ROM.

In the above described example, two particular disease groups, i.e. chronic obstructive pulmonary disease (COPD) and asthma, were considered. Other disease groups are of course envisaged.

## Claims

1. A system for use in a network and for storing data imported from one or more distinct storage devices of the network in a database, comprising:
a first module configured to:
search data stored in a first storage device comprising a memory storing a data bank comprising sets of data associated with users, the search being performed as a function of criteria to identify at least a user and an associated set of data, both corresponding to the search criteria,
remove personal information about the identified user from the set of data to generate an anonymous set of data,
allocate a first identifier to the user, and
link the anonymous set of data to the first identifier;
a second module configured to:
import the anonymous set of data and the first identifier from the first storage device,
generate an anonymous set of requests for information to be sent to the identified user, as a function of the anonymous set of data, and
link the anonymous set of requests for information to the first identifier,
provide the anonymous set of requests for information to an output device,
import an anonymous set of responses from responses to the anonymous set of requests sent back by the user through a second storage device comprising a memory, and
link the anonymous set of responses to the first identifier,
the system further comprising a database, and
wherein the second module is further configured to provide:
the anonymous set of data and the first identifier from the first storage device and the anonymous set of responses from the second storage device to the database, and
the database is further configured to store the anonymous set of data and the anonymous set of responses, and link them to the first identifier.

2. The system according to claim 1, wherein the second module is further configured to:
generate an anonymous status as a function of at least the anonymous set of data and the anonymous set of responses from the database, and link the anonymous status to the first identifier, and
generate a summary as a function of a plurality of anonymous statuses from the database, and
wherein the database is configured to store the anonymous status and the summary;
optionally wherein the second module is further configured to:
provide the anonymous status and the summary to the first storage device, and
the first module is configured to:
link back the anonymous status to the identified user thanks to the first identifier, to generate a personalized status.

3. The system according to any one of claims 1 or 2, wherein the database is further configured to:
allocate a second identifier to the first identifier in a first database, and
store the second identifier, the anonymous set of data and the anonymous set of responses in a second database, the database linking the anonymous set of data and the anonymous set of responses to the first identifier via the second identifier, or
wherein the first module is further configured to provide personalized information about the identified user to the output device, optionally wherein the output device comprises a printer configured to print:
the anonymous set of requests for information to be sent to the identified user as a questionnaire comprising the first identifier, and
the personalized information as a cover letter comprising pieces of personal information about the identified user,
optionally wherein the second storage device comprises a scanner configured to scan the anonymous set of responses, from the identified user and comprising the first identifier, as a questionnaire filled in by the identified user, using an optical character recognition software.

4. The system according to any one of claims 1 to 3, wherein the output device comprises at least one of a printer and a server, or wherein the second storage device comprises at least one of a scanner and a server, or wherein the output device further comprises the second storage device and comprises a server which is configured to be accessed by a graphical user interface to display the anonymous set of requests to the identified user and to enable the identified user to enter the set of responses to the anonymous set of requests.

5. The system according to any one of claims 1 to 4, wherein the second module is further configured to:
import a set of result data regarding measures concerning the identified user from a third storage device, and link the set of result data to the first identifier, and
provide the set of result data from the third storage device to the database, and
wherein the database is further configured to store the set of result data, and link them to the first identifier.

6. The system according to claim 5, wherein the third storage device comprises an adapter to an inhaler belonging to the identified user and/or an acceleration tester.

7. The system according to any one of claims 1 to 6, wherein the second module is configured to perform a verification of the imported data from the first device, or wherein the second module is configured to perform a case-control matching on the data stored on the database.

8. A method performed by a system in a network, for importing data from one or more distinct storage devices of the network, and for storing the imported data in a database, the method comprising the steps according to which:
a first module
searches data stored in a first storage device comprising a memory storing a data bank comprising sets of data associated with users, the search being performed as a function of criteria to identify at least a user and an associated set of data, both corresponding to the search criteria,
removes personal information about the identified user from the set of data to generate an anonymous set of data,
allocates a first identifier to the user, and links the anonymous set of data to the first identifier;
a second module:
imports the anonymous set of data and the first identifier from the first storage device,
generates an anonymous set of requests for information to be sent to the identified user, as a function of the anonymous set of data, and links the anonymous set of requests for information to the first identifier,
provides the anonymous set of requests for information to an output device,
imports an anonymous set of responses from responses to the anonymous set of requests sent back by the user through a second storage device comprising a memory, and
links the anonymous set of responses to the first identifier,
the method further comprising the step wherein the second module further provides:
the anonymous set of data and the first identifier from the first storage device, and
the anonymous set of responses from the second storage device to a database, and the database stores the anonymous set of data and the anonymous set of responses, and links them to the first identifier.

9. The method according to claim 8, wherein the second module further:
generates an anonymous status as a function of at least the anonymous set of data and the anonymous set of responses from the database, and links the anonymous status to the first identifier, and
generates a summary as a function of a plurality of anonymous statuses from the database, and
wherein the database stores the anonymous status and the summary, optionally wherein the second module further provides the anonymous status and the summary to the first storage device, and the first module links back the anonymous status to the identified user thanks to the first identifier, to generate a personalized status.

10. The method according to any one of claims 8 or 9, wherein the database further allocates a second identifier to the first identifier in a first database, and stores the second identifier, the anonymous set of data and the anonymous set of responses in a second database, the database linking the anonymous set of data and the anonymous set of responses to the first identifier via the second identifier, or wherein the first module further provides personalized information about the identified user to the output device.

11. The method according to any one of claims 8 to 10, wherein the output device comprises a printer which prints:
the anonymous set of requests for information to be sent to the identified user as a questionnaire comprising the first identifier, and
the personalized information as a cover letter comprising pieces of personal information about the identified user, optionally wherein the second storage device comprises a scanner which scans the anonymous set of responses, from the identified user and comprising the first identifier, as a questionnaire filled in by the identified user, using an optical character recognition software.

12. The method according to any one of claims 8 to 11, wherein the output device further comprises the second storage device and comprises a server which is accessed by a graphical user interface to display the anonymous set of requests to the identified user and to enable the identified user to enter the set of responses to the anonymous set of requests, or wherein the second module further:
imports a set of result data regarding measures concerning the identified user from a third storage device, and links the set of result data to the first identifier, and
provides the set of result data from the third storage device to the database, and
wherein the database further stores the set of result data, and links them to the first identifier.

13. The method according to any one of claims 8 to 12, wherein the second module performs a verification of the imported data from the first device, or wherein the second module performs a case-control matching on the data stored on the database, optionally wherein the system:
identifies matching criteria;
creates a combined database file containing records for patient cases, corresponding control patients and identified matching criteria variables;
randomly selects from the combined database file a case-control pair, where a case matching criteria variables match a control matching criteria variables, and
writes the case control pair record in a matching table file,
selects a case-control matching ratio, and randomly selects a case identifier and
randomly selects at least a control identifier matched to the case identifier from the matching table file; and
writes the matched case and control in a holding data table file.

14. The method according to any one of claims 8 to 13, wherein the search criteria to identify at least a user and an associated set of data is the matching with a particular disease group comprising at least one of chronic obstructive pulmonary disease, COPD, and asthma, or wherein the user is a patient registered with a general practitioner, and the sets of data are clinical data records.

15. A computer program, a signal, a computer program product or a computer readable medium comprising instructions for carrying out a method according to any one of claims 8 to 14.
